# EUROPÄISCHE PATENTANMELDUNG

(11) **EP 3 666 248 A1**
(43) Veröffentlichungstag der Anmeldung: **17.06.2020**
(21) Anmeldenummer: 19208140.4
(22) Anmeldetag: 08.11.2019
(51) Int. Cl.: A61K 8/41, A61K 8/81, A61Q 5/12

(54) **PFLEGENDE HAAR-STYLING-EMULSION**

(30) Priorität: 13.12.2018 DE 102018221615
(71) Anmelder: Beiersdorf AG, 20253 Hamburg (DE)
(72) Erfinder: Reiter, Katharina, 21357 Barum (DE); Nemnich, Julia, 22589 Hamburg (DE)

(57) **Zusammenfassung**

Haarzubereitung zur Haarpflege und zum Haar-Styling, wobei die Haare einen Hitzeschutz erhalten.

## Beschreibung

Die vorliegende Erfindung liegt auf dem kosmetischen Gebiet von Haarzubereitungen und zwar auf dem Gebiet der Haarpflege und Haar-Styling-Zubereitungen. Die erfindungsgemäßen Zubereitungen zeichnen sich dadurch aus, dass sie einen Hitzeschutz für das Haar bereitstellen, der bewirkt, dass das Haar bei Hitzeanwendungen, z.B. Föhnen, Glätten und anderen mehr, geschützt ist.

Das Ziel von Haarpflegezubereitungen ist es, dazu beizutragen, dass das Haar natürlich und gepflegt aussieht.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt das Haar eine soziale Funktion, da es über sein Erscheinungsbild erheblich zu zwischenmenschlichen Beziehungen und zum Selbstwertgefühl des Individuums beiträgt.

Das Haar besteht aus einem Haarschaft, der frei aus der Haut herausragt. Der Haarschaft ist keratinisiert (tot) und ist der sichtbare Teil des Haares. Die nicht-sichtbare Haarwurzel ist der lebende Teil und in der Haut lokalisiert. In der Haarwurzel wird das Haar gebildet. Der Haarschaft ist aus drei Schichten aufgebaut: einem zentralen Teil, dem so genannten Haarmark (Medulla), das beim Menschen allerdings zurückgebildet ist und oft gänzlich fehlt, dem Mark (Cortex) und der äußeren, bis zu zehn Lagen starken, Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Ziel der Haarpflege ist es, das Haar zu unterstützen, den Zustand des frisch nach gewachsenen Haares möglichst lange zu erhalten und/oder Haarschädigungen entgegen zu wirken.

Bekannt sind viele Möglichkeiten, das Haar zu pflegen. Von diesen Möglichkeiten ist die Verwendung eines Conditioner-Produktes nach der Haarwäsche weit verbreitet und sehr beliebt. Diese Conditioner-Produkte enthalten sogenannte konditionierende Verbindungen, die mit der Haaroberfläche wechselwirken. Die Hauptgruppen dieser konditionierenden Verbindungen sind quaternäre Ammonium-Verbindungen, Fette und Öle in geringen Mengen, Silikonverbindungen und schwache Säuren. Die Conditioner-Produkte werden jedoch wieder ausgespült. Durch dieser Vorgang gehen dem Haar konditionierende Verbindungen wieder verloren. Hierdurch ist die Pflegewirkung nicht so optimal wie gewünscht.

Beliebt beim Verbraucher ist die Verwendung von Haarstyling-Zubereitungen. Diese Zubereitungen werden auf das Haar aufgetragen und bleiben auf dem Haar. Die so behandelten Haare können jetzt gestylt werden, so dass eine gewünschte Frisur erhalten wird oder die Styling-Produkte werden auf das gestylte Haar aufgetragen und durch die Styling-Produkte fixiert. Grob lassen sich zwei Formen von Haarstyling-Zubereitungen unterscheiden, zum einen Schaumfestiger, auch manchmal als Fönfestiger bezeichnet, und Haarsprays. Die Hauptkomponente in beiden Zubereitungsformen sind Filmbildner, die bisweilen auch als festigende Polymere bezeichnet werden. Die Filmbildner können sich auf die Oberfläche eines einzelnen Haares legen und kleine Brücken zum Film eines benachbarten Haares ausbilden. Auf diese Weise können die Haare in der gewünschten Weise geformt werden und diese Form wird durch den Film und die Brücken zwischen den Haaren stabilisiert. Die Styling-Zubereitungen bleiben im Haar und werden erst bei der nächsten Haarwäsche wieder ausgewaschen. Obwohl es in der Vergangenheit Versuche gegeben hat, Haarstyling-Zubereitungen auch pflegende Eigenschaften zu verleihen, liegt der Hauptschwerpunkt der derzeit auf dem Markt befindlichen Haarstyling-Zubereitungen eindeutig auf der Erzielung der Stylingwirkung.

Im Stand der Technik gibt es viele Dokumente zu Conditioner-Zubereitungen und Styling-Zubereitungen. Beispielhaft seien die folgenden genannt:
Im Dokument EP 2358442 B1 werden konditionierende Zubereitungen beschrieben, die in Form einer W/O-Emulsion vorliegen und Silikontenside enthalten.

Das Dokument WO 2017/165112 A1 offenbart klare Conditioner-Zubereitungen, die ebenfalls Silikon-Tenside enthalten, zusätzlich aber kationische Polymere, die auch als Verdicker wirken.

Im Dokument EP 1028698 B1 werden Styling-Zubereitungen beschrieben, die Silikon-Emulsionen enthalten.

Das Dokument WO 2018/018488 A1 offenbart Styling-Zubereitungen mit nichtionischen Styling-Polymeren und kationischen Polymeren, die zusätzlich einen Hagebutten-Extrakt enthalten.

Es bestand nun der Wunsch beide Produktformen zu vereinen und ein Produkt zur Verfügung zu stellen, das die Pflegeleistung eines Conditioner mit der Festigungswirkung einer Styling-Zubereitung verbindet. Auf diese Weise kann in der Haarbehandlungsroutine ein Schritt eingespart werden, nämlich anhaltende Pflege und Styling in einem Schritt. Es ist aber auch möglich, dieses neue Produkt als zusätzliche Produktform zu verwenden, die nach dem Ausspülen des Shampoos und/oder Conditioners auf das Haar aufgetragen wird. Diese Produktform verstärkt die Pflegewirkung des Conditioners, weil sie nicht mehr ausgespült wird und die konditionierenden Komponenten im Haar bleiben. Gleichzeitig wird die Wirkung des Haarstyling-Produktes verstärkt, weil die neue Produktform festigende Eigenschaften hat. Somit bestand die Aufgabe der vorliegenden Erfindung eine Produktform zur Verfügung zu stellen, die konditionierende und festigende Eigenschaften in einer Zubereitung vereint. Insbesondere sollte diese Produktform die Haare bei Hitzeeinwirkung schützen.

Überraschend konnten die Aufgaben gelöst werden durch die Bereitstellung einer Zubereitung, die
eine Kombination quaterärer Ammoniumverbindungen ausgewählt aus
- wenigstens einem kationischen Tensid in Form eines Esterquats und
- wenigstens einem kationischen Polymer gemäß folgender Strukturformel 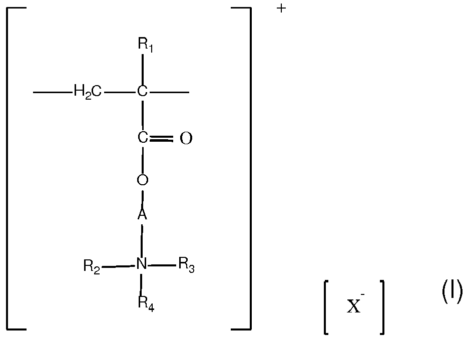
wobei R₁ -CH₃, A -(CH₂)₂, und R₂, R₃ und R₄ jeweils -CH₃ und X⁻ Cl⁻ entsprechen und wenigstens ein nichtionisches fixierendes Polymer, enthaltend Vinylpyrolidone-Monomere,
enthält.

Die Zubereitung ist eine kosmetische Zubereitung, die vorteilhaft auf das menschliche Kopfhaar aufgetragen wird und nicht wieder ausgespült wird (leave-on). Weiter vorteilhaft liegt die erfindungsgemäße Zubereitung in Form einer Emulsion vor, insbesondere einer O/W-Emulsion. Die erfindungsgemäße Zubereitung ist eine wässrige Zubereitung.

Die erfindungsgemäße Zubereitung enthält wenigstens ein kationisches Tensid, ausgewählt aus der Gruppe der Esterquats. Esterquats zeichnen sich durch zwei hydrophobe Gruppen im Molekül aus, die über Esterbindungen mit einem quaternisierten Di(Tri-)ethanolamin verknüpft sind. Esterquat haben den Vorteil, dass sie biologisch besser abbaubar sind als kationische Tenside, die keine Esterbindung aufweisen.

Bevorzugt werden in der erfindungsgemäßen Zubereitung Esterquats verwendet, die folgende allgemeine Strukturformel haben: 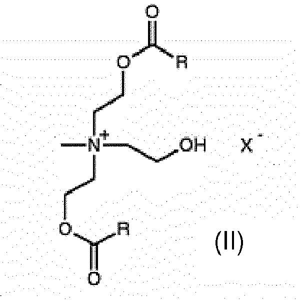 wobei R einem organischen Rest mit 10 bis 30, bevorzugt 12 bis 24 Kohlenstoffatomen entspricht, insbesondere ein substituierter oder unsubstituierter, verzweigter oder unverzweigter Alkylrest, der auch ungesättigt sein kann, wobei der Alkylrest bevorzugt ein linearer gesättigter Alkylrest mit 14 bis 20 Kohlenstoffatomen ist. Das Gegenion X⁻ wird bevorzugt aus der Gruppe Chlorid, Bromid und Methosulfat ausgewählt. Ganz besonders bevorzugt sind die Verbindungen Dipalmitoylethyl Hydroxyethylmonium Methosulfate und Distearoylethyl Hydroxyethylmonium Methosulfate, wobei Distearoylethyl Hydroxyethylmonium Methosulfate am meisten bevorzugt ist.

Distearoylethyl Hydroxyethylmonium Methosulfate kann beispielsweise als Dehyquart F 75 T bei der Firma BASF bezogen werden.

Das wenigstens eine Esterquat ist in der erfindungsgemäßen Zubereitung mit einem Gehalt von 0,01 bis 10 Gew.-%, bevorzugt 0,1 bis 7,0 Gew.-% und besonders bevorzugt 0,2 bis 5,0 Gew.-% enthalten, bezogen auf das Gesamtgewicht der Zubereitung und bezogen auf den Aktivgehalt.

Die erfindungsgemäße Zubereitung enthält wenigstens ein kationisches Polymer gemäß Strukturformel (I). Das kationische Polymer mit der INCI-Bezeichnung Polyquaternium-37 ist ein Polymer gemäß Strukturformel (I). Polyquaternium-37 ist erfindungsgemäß bevorzugt und kann mit der Handelsbezeichnung Flocare PDS 1037 bei der Firma SNF bezogen werden.

Das wenigstens eine kationische Polymer gemäß Strukturformel (I) ist in der erfindungsgemäßen Zubereitung mit einem Gehalt von 0,1 bis 8 Gew.-%, bevorzugt 0,2 bis 7.0 Gew.-% und besonders bevorzugt 0,4 bis 5,0 Gew.-% enthalten, bezogen auf das Gesamtgewicht der Zubereitung und bezogen auf den Aktivgehalt.

Die erfindungsgemäße Zubereitung enthält wenigstens ein nichtionisches fixierendes Polymer, enthaltend Vinylpyrolidone-Monomere. Bevorzugt können diese Polymere ausgewählt werden aus der Gruppe der Homopolymere des Vinylpyrolidons und der Gruppe der Copolymere des Vinylpyrrolidons wie Copolymere aus Vinylpyrrolidon und Vinylacetat (VP/VA), Terpolymere aus, Polyvinylamide und deren Salze, Copolymere aus Vinylpyrrolidon und Dimethylaminoethylmethacrylat, Terpolymere aus Vinylcaprolactam, Vinylpyrrolidon und Dimethylaminomethacrylat. Besonders bevorzugt sind Copolymere aus Vinylpyrolidone und Vinylacetat, die unter der Handelsbezeichnung Luviskol VA 64 W bei der Firma BASF erhältlich sind.

Das wenigstens eine nichtionische fixierende Polymer, enthaltend Vinylpyrolidone-Monomere ist in der erfindungsgemäßen Zubereitung mit einem Gehalt von 0,01 bis 10,0 Gew.-%, bevorzug 0,1 bis 7,0 Gew.-% und besonders bevorzugt 0,2 bis 5,0 Gew.-% enthalten, bezogen auf das Gesamtgewicht der Zubereitung und bezogen auf den Aktivgehalt.

Darüber hinaus können vorteilhaft weitere Komponenten in der erfindungsgemäßen Zubereitung enthalten sein.

Vorteilhaft ist die Einarbeitung von Ölkomponenten. Diese Ölkomponenten können ausgewählt werden aus der Gruppe der Ester aus gesättigten und/oder ungesättigten, verzweigten und/oder unverzweigten Alkancarbonsäuren einer Kettenlinie von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat.

Ferner kann die Ölkomponente vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlaenge von 8 bis 24, insbesondere 12 bis 18 C-Atomen. Die Fettsäuretriglyceride können beispielsweise vorteilhaft gewählt werden aus der Gruppe der natürlichen Öle, z. B. Olivenöl, Sonnenblumenöl, Sojaöl, Erdnussöl, Rapsöl, Mandelöl, Palmöl, Kokosöl, Palmkerne und dergleichen mehr.

Es ist bevorzugt, wenn wenigstens ein gesättigter oder ungesättigter, verzweigter oder unverzweigter Alkohol in der erfindungsgemäßen Zubereitung enthalten ist. Besonders bevorzugt ist es, wenn wenigstens ein gesättigter und unverzweigter Alkohol mit einer Kettenlänge von 10 bis 24 Kohlenstoffatomen enthalten ist, weiter besonders bevorzugt ist es, wenn der wenigstens eine Alkohol aus der Gruppe Stearylalkohol, Cetylalkohol und/oder der Mischform Cetearylalkohol ausgewählt wird.

Die wenigstens eine Ölkomponente ist in der erfindungsgemäßen Zubereitung mit einem Gehalt von 0,1 bis 10,0 Gew.-%, bevorzugt 0,2 bis 6,0 Gew.-% und besonders bevorzugt 0,25 bis 4,0 Gew.-% enthalten, bezogen auf das Gesamtgewicht der Zubereitung.

Zusätzlich kann wenigstens eine Silikonverbindung in der erfindungsgemäßen Zubereitung enthalten sein. Silikone haben auch die chemische Bezeichnung Poly(organo)siloxane. Es handelt sich um eine Gruppe synthetischer Polymere, bei denen Siliziumatome über Sauerstoffatome verknüpft sind. Silikone bestehen aus einzelnen Siloxaneinheiten, die aus Siliciumatomen, verbunden über eine Sauerstoffbrücke, bestehen.

Die allgemeine Formel lautet RₙSiO_{(4-n)/2} (n=0, 1, 2, 3), d. h. eine Siloxaneinheit kann ein bis vier weitere Substituenten aufweisen.

Siloxaneinheiten können also mono-, di-, tri- und tetrafunktionell sein. In symbolischer Schreibweise stellt man dies durch folgende Buchstaben dar:
M (mono), M entspricht R₃SiO_{1/2},
D (di), D entspricht R₂SiO_{2/2},
T (tri), T entspricht RSiO_{3/2} und
Q (quatro), Q entspricht SiO_{4/2}.

Wie bei den organischen Polymeren basiert die Vielzahl der möglichen Verbindungen darauf, dass verschiedene Siloxaneinheiten im Molekül miteinander verknüpft werden können. Angelehnt an die Systematik der organischen Polymere kann man folgende Gruppen unterscheiden:
- Cyclische Polysiloxane sind ringförmig aus difunktionellen Siloxaneinheiten aufgebaut, Dₙ.
- Lineare Polysiloxane sind folgendermaßen aufgebaut MDₙM, bzw. R₃SiO[R₂SiO]ₙSiR₃, als Beispiel sei Polydimethylsiloxan genannt.
- Vernetzte Polysiloxane sind ketten- oder ringförmige Moleküle, die mit Hilfe von tri- und tetrafunktionellen Siloxaneinheiten zu ebenen oder dreidimensionalen Netzwerken verknüpft sind. Der Aufbau hochmolekularer Silikone erfolgt über Kettenbildung und Vernetzung.
- Verzweigte Polysiloxane weisen als verzweigende Elemente trifunktionelle oder tetrafunktionelle Siloxaneinheiten auf, MₙDₘTₙ. Die Verzweigungsstellen sind dabei entweder in eine Kette oder einen Ring eingebaut.

Am Siliciumatom der Silikone können unterschiedliche Substituenten gebunden sein. Diese Substituenten haben Einfluss auf die Eigenschaften der entsprechenden Silikonverbindungen.

Es ist erfindungsgemäß vorteilhaft, wenn die Silikonverbindungen lineare Abschnitte aufweisen und die Substituenten Alkoxylreste sind. Bevorzugt ist es, wenn diese Alkoxyreste sowohl Ethylen Glycol-Reste als auch Propylen-Glycol-Reste enthalten, insbesondere aus diesen Resten aufgebaut sind. Die entsprechenden Silikonverbindungen haben oft oberflächen- oder grenzflächen-aktive Eigenschaften und werden deshalb dann auch als Silikontenside bezeichnet. Besonders bevorzugt ist es, wenn die erfindungsgemäßen Zubereitungen PEG/PPG-18/18 Dimethicone enthalten, das als Xiameter OFX-5220 Fluid von der Firma Dow Corning erhältlich ist.

Die wenigstens eine Silikonverbindung ist in der erfindungsgemäßen Zubereitung mit einem Gehalt von 1,0 bis 7,0 % Gew.-%, bevorzugt 0,2 bis 5,0 % Gew.-% und besonders bevorzugt 0,5 bis 3,0 % Gew.-%, bezogen auf das Gesamtgewicht der Zubereitung und bezogen auf den Aktivgehalt.

Zusätzlich kann wenigstens ein weiteres kationisches Tensid in der erfindungsgemäßen Zubereitung enthalten sein. Das weitere kationische Tensid ist kein Esterquat. Es ist bevorzugt, wenn das wenigstens eine weitere kationische Tensid ausgewählt wird aus der Gruppe der Amidoamine. Diese Verbindungen können erhalten werden durch die Reaktion von Fettsäuren mit Diaminen und nachfolgender Quaternierung der freien Aminfunktion. Es ist weiter bevorzugt, wenn das weitere kationische Tensid ausgewählt wird aus der Gruppe Stearylamidopropyltrimmonium Chloride, Ricinoleamidopropyltrimonnium Chloride und/oder Palmitamidopropyltrimonium Chloride; am meisten bevorzugt ist es, wenn Palmitamidopropyltrimonium Chlorid enthalten ist. Palmitamidopropyltrimonium Chlorid kann als Varisoft PATC bei der Firma Evonik Industries bezogen werden.

Ebenso bevorzugt ist es, wenn das wenigstens eine weitere kationische Tensid aus der Gruppe der Ammoniumsalze gewählt wird. Weiter bevorzugt ist es, wenn das wenigstens eine weitere kationische Tensid aus der Gruppe der primären Ammoniumsalze gewählt wird, die nur einen langkettigen organischen Rest aufweisen. Besonders bevorzugt ist es, wenn das wenigstens eine weitere kationische Tensid ausgewählt wird aus der Gruppe Cetyltrimethylammoniumchlorid (Cetrimonium Chlorid), Stearyltrimethylammoniumchlorid und/oder Behentrimoniumchlorid. Am meisten bevorzugt ist es, wenn das wenigstens eine weitere kationische Tensid Cetrimonium Chlorid ist.

Das wenigstens eine weitere kationische Tensid ist in der erfindungsgemäßen Zubereitung mit einem Gehalt von 0.1 bis 4 Gew.-%, bevorzugt 0.2 bis 2 Gew.-% enthalten, bezogen auf das Gesamtgewicht der Zubereitung und bezogen auf den Aktivgehalt. Im Sinne der vorliegenden Erfindung ist der Gehalt des wenigstens einen weiteren kationischen Tensids nicht im Gehalt der kationischen Tenside ausgewählt aus der Gruppe der Esterquats enthalten.

Zusätzlich kann wenigstens ein weiteres kationische Polymer in der erfindungsgemäßen Zubereitung enthalten sein. Das wenigstens eine weitere kationische Polymer ist kein Polymer gemäß der Strukturformel (I). Dieses Polymer kann synthetischen oder natürlichen Ursprungs sein. Es kann weiterhin unterschieden werden zwischen permanent und temporär kationischen Polymeren. Die permanent kationischen Polymere zeichnen sich durch Ammoniumgruppen aus, die dauerhaft eine positive Ladung tragen. Die temporär kationischen Polymere weisen amino-funktionelle Gruppen auf, die im saureren Milieu ein Proton anlagern können und dann positiv geladen sind, während bei basischeren pH-Werten kein Proton angelagert ist und somit keine positive Ladung vorhanden ist.

Ein kationisches Polymer mit permanent positiver Ladung ist beispielsweise Polyquaternium 7. Dies ist ein Beispiel für ein derartiges Polymer synthetischen Ursprungs.

Kationische Polymere mit permanent positiver Ladung natürlichen Ursprungs sind beispielsweise quaternisierte Cellulose-Derivate, wie beispielsweise Polyquaternium 10 oder kationische Guar-Derivate, wie beispielsweise Guar Hydroxypropyltrimonium Chloride. Es ist bevorzugt, wenn Guar Hydroxypropyltrimonium Chloride in der erfindungsgemäßen Zubereitung enthalten ist. Guar Hydroxypropyltrimonium Chloride kann beispielsweise unter der Handelsbezeichnung Jaguar C-14 S bei der Firma Solvay bezogen werden.

Das wenigstens eine weitere kationische Polymer liegt in der erfindungsgemäßen Zubereitung mit einem Gehalt von 0,01 bis 1,0 Gew.-%, bevorzugt 0,05 bis 0,75 Gew.-% vor, bezogen auf das Gesamtgewicht der Zubereitung und bezogen auf den Aktivgehalt. Im Sinne der vorliegenden Erfindung ist der Gehalt des wenigstens einen weiteren kationischen Polymers nicht im Gehalt des wenigstens einen kationischen Polymers aufweisend eine Struktur gemäß Strukturformel (I) enthalten.

Zusätzlich kann wenigstens eine organische Säure enthalten sein. Die organischen Säuren werden benötigt, um den pH-Wert der Zubereitung auf einen sauren pH-Wert im physiologischen Bereich einzustellen. Vorteilhaft ist ein pH-Wert-Bereich von 4,0 bis 5,5.

Es ist vorteilhaft, wenn Citronensäure und/oder Milchsäure ausgewählt werden, um den pH-Wert einzustellen.

Weiterhin ist es vorteilhaft, wenn die Zubereitung ein Parfüm enthält. Parfüme sind Mischungen aus Parfümrohstoffen. Das Parfüm liegt vorteilhaft mit einem Gehalt von 0.05 bis 1 vor, bezogen auf das Gesamtgewicht der Zubereitung.

Ebenso ist es vorteilhaft, wenn die erfindungsgemäße Zubereitung Konservierungsmittel enthält. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können vorteilhaft Phenoxyethanol, Methyl-, Ethyl-, Propylparaben, Benzylalkohol, Sorbinsäure und/oder Salze der Sorbinsäure, beispielsweise Kalium Sorbat, Benzoesäure und/oder Salze der Benzoesäure, beispielsweise Natrium Benzoat, Salicylsäure und/oder Salze der Salicylsäure, beispielsweise Natrium Salicylat eingesetzt werden. Die genannten Konservierungsmittel können einzeln oder in Kombination eingesetzt werden.

Als Stabilisatoren sind im Sinne der vorliegenden Erfindung diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung haben und/oder im gemeinsamen Einsatz mit Konservierungsmitteln die Stabilität der Zubereitung fördern.

Als Stabilisatoren sind folgende Verbindungen vorteilhaft: Alcohol Denat., Propylene Glycol, Ethylhexylglycerin, 1,2-Hexanediol, Trisodium EDTA, Methylpropanediol, Butylene Glycol, Caprylyl Glycol, Piroctone Olamine und Pentylene Glycol. Die genannten Stabilisatoren können einzeln oder in Kombination eingesetzt werden.

Die Stabilisatoren, einzeln oder mehrere, werden bevorzugt zusammen mit einem oder mehreren Konservierungsmitteln verwendet.

Das wenigstens eine Konservierungsmittel und/oder der wenigstens eine Stabilisator liegen mit einem Gehalt von 0,0001 bis 10 Gew. %, bevorzugt von 0,0005 bis 8 Gew. %, besonders bevorzugt von 0,001 bis 7 Gew. % in der erfindungsgemäßen Zubereitung vor, bezogen auf das Gesamtgewicht der Zubereitung.

Die erfindungsgemäße Zubereitung kann auf jede für eine derartige Zubereitung im StdT bekannte und geeignete Weise hergestellt werden Bevorzugt ist es jedoch, wenn die folgende Methode angewendet wird: Das Wasser und die im Wasser löslichen Rohstoffe werden gemischt und auf 75°C erhitzt. Anschließend werden die fettlöslichen Rohstoffe hinzugegeben und aufgeschmolzen. Anschließend wird das kationische Polymer gemäß Strukturformel (I) hinzugegeben und das Produkt homogenisiert. Nach Abkühlen auf unter 60°C wird das nichtionische fixierende Polymer hinzugegeben. Nach Abkühlen auf etwa 35°C wird das Parfüm hinzugegeben und nach Abkühlen auf etwa 30°C wird das das Produkt erneut homogenisiert.

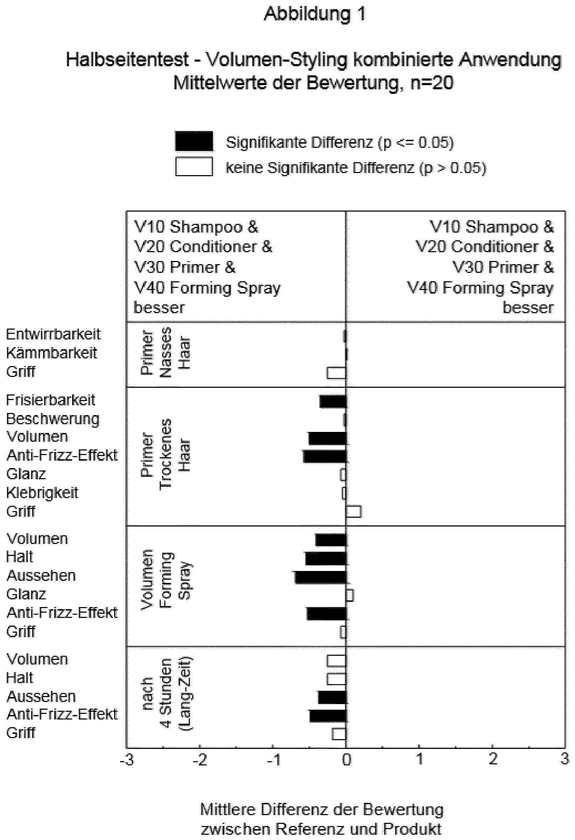

Um zu belegen, dass ein Produkt gemäß des Ausführungsbeispiels 4 die Aufgaben löst, die dieser Erfindung zugrunde lagen, wurde ein "half-head test" durchgeführt. Dazu wurden bei 20 Frauen (Alter 26-71 Jahre; Haarlänge: Kurz bis mittellang; Haardicke: Fein bis normal) ein Testshampoo (je nach Haarlänge 10 - 14 ml) und ein Test-Konditioner (je nach Haarlänge 12 - 16 g) verwendet. Dann wurden die Haare in zwei Hälften unterteilt, auf die eine Hälfte wurde das erfindungsgemäße Produkt gemäß Ausführungsbeispiel 4 (in Abbildung 1 als Primer bezeichnet) aufgetragen (0,7 bis 1,0 g je nach Haarlänge), auf die andere Haarhälfte wurde nichts aufgetragen. Während des Auftrags wurden die Parameter Konsistenz, Verteilbarkeit und Klebrigkeit beurteilt. Diese Beurteilung und alle weiteren Beurteilungen erfolgten durch 2 Frisöre, die in derartigen Beurteilungen ausgebildet sind und bereits viel Erfahrung bei derartigen Beurteilungen aufweisen. Nach dem Auftrag des erfindungsgemäßen Produktes wurden für beide Haarhälften die Parameter Entwirrbarkeit, Kämmbarkeit und wie sich das Haar anfühlt (hair feel) beurteilt. Dann wurden die Haare mithilfe eines Föns getrocknet und dabei gestylt. Nach diesem Schritt wurden die Parameter Handhabbarkeit (manageability), Nicht-Fettigkeit (non-greasiness), Volumen, Anti-Kräuselungseffekt (anti-frizz effect), Glanz, Klebrigkeit und wie sich das Haar anfühlt (hair feel) für beide Haarhälften beurteilt. Danach wurde ein Volumen-Haarspray auf beide Haarhälften aufgetragen und dann die Parameter Volumen, Halt, Aussehen (look), Glanz, Anti-Kräuselungseffekt (anti-frizz effect) und wie sich das Haar anfühlt (hair feel) bestimmt. Nach 4 bis 6 Stunden wurden noch einmal die Parameter Volumen, Halt, Aussehen (look), Glanz, Anti-Kräuselungseffekt (anti-frizz effect) und wie sich das Haar anfühlt (hair feel) beurteilt.

Die Ergebnisse sind in Abbildung 1 dargestellt. Schwarze Balken zeigen signifikante Unterschiede in der Beurteilung, graue Balken zeigen Unterscheide. Primer bedeutet das erfindungsgemäße Produkt. Es wird deutlich, dass die Verwendung des erfindungsgemäßen Produktes zu besseren Beurteilungen in der Handhabbarkeit, im Volumen und im Anti-Kräuseleffektes (anti frizz effect) im trockenen Haar führt. Ebenso sind die Beurteilungen in Bezug auf Volumen, Halt, Aussehen (look) und Anti-Kräuselungseffekt (anti-frizz effect) nach Anwendung des Volumen-Haarsprays für die Haarhälfte, die mit dem erfindungsgemäßen Produkt behandelt wurde, signifikant besser als für die Haarhälfte, die mit diesem Produkt nicht behandelt wurde. Dieser Effekt ist auch nach 4 bis 6 Stunden noch vorhanden. Insgesamt lässt sich aus diesen Ergebnissen ableiten, dass das erfindungsgemäße Produkt zu Verbesserungen bei pflegenden, konditionierenden Parametern (Handhabbarkeit, Volumen und Anti-Kräuselungseffet (anti-frizz effect) führen, aber genauso zu Verbesserungen der Stylingeigenschaften, wie Halt und Aussehen (look). Dies belegt, dass die Aufgaben, die der Erfindung zugrunde lagen, gelöst wurden.

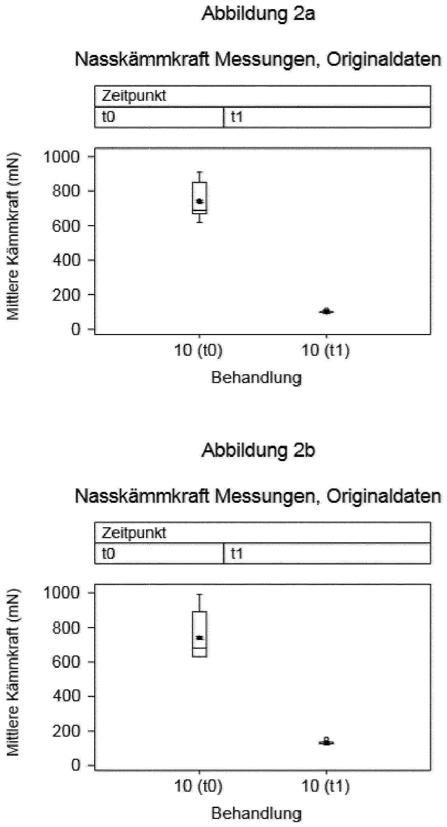

Darüber hinaus wurde in einem *in vitro* Test die Nasskämmbarkeit untersucht. Hierzu wurde ein Produkt gemäß Ausführungsbeispiel 4 auf jeweils 7 Haarsträhnen (Haarsträhne von je 2g, geschädigt) aufgetragen. Zuvor wurden die Haarsträhnen mit einem Testshampoo gewaschen und das erste Mal in Bezug auf die Nasskämmbarkeit und die dabei einzusetzende Kraft vermessen. Dann wurde die Messung nach Anwendung des erfindungsgemäßen Produktes wiederholt. Die Ergebnisse sind in Abbildungen 2a und 2b dargestellt. In Abbildung 2a ist auf der y-Achse die mittlere Kraft aufgetragen, in Abbildung 2b die maximale Kraft. Aus beiden Abbildungen lässt sich ablesen, dass nach Verwendung des erfindungsgemäßen Produktes weniger Kraft für das Kämmen nötig ist. Wenn weniger Kraft nötig ist, ist das Kämmen einfacher, d.h. die Oberfläche des Haares ist glatter. Dies ist ein Beleg für einen guten konditionierenden Effekt des erfindungsgemäßen Produktes.

Weiterhin wurde in einem *in vitro* Test untersucht, ob das erfindungsmäße Produkt einen Hitzeschutz ausübt. Dazu wurden jeweils 5 Haarsträhnen (2 g, ungeschädigtes Haar) für 15 Minuten in Wasser gelegt und mit Test-Shampoo gewaschen. 5 Haarsträhnen wurden Luft getrocknet (ungeschädigte Haarsträhnen, Referenzwerte, Code 01).

Jeweils 5 Haarsträhnen wurden nach dem Auftrag des Test-Shampoos und dem Auswaschen des Shampoos mit dem Fön getrocknet und mit einem Kamm geglättet. Anschließend folgte eine Hitzebehandlung; dabei wurde die Strähne mit gleichmäßiger Geschwindigkeit durch ein Flacheisen (235°C) gezogen, 20 Sekunden abgekühlt, gedreht. Dieser Schritt wurde neunmal wiederholt (also insgesamt 10 Hitzeschritte). Die Strähnen wurden mit Wasser gewaschen und dann mit dem Test-Shampoo behandelt. Dieser Ablauf, 10 Hitzeschritte und ein Waschschritt, wurden 5mal wiederholt. Anschließend wurden die Strähnen ausgewaschen. Diese Strähnen sind die unbehandelten Strähnen, Code 05.

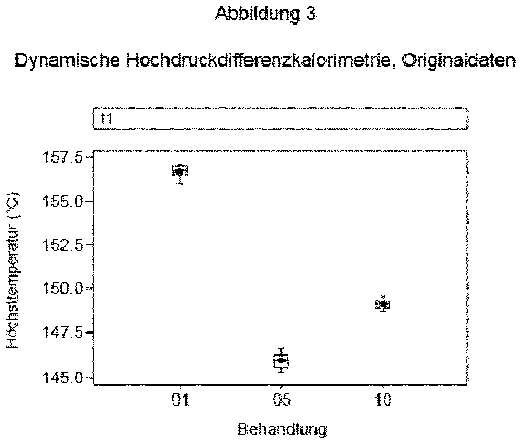

Für die behandelten Haarsträhnen (Code 10) wurde nach dem ersten Auftrag des Test-Shampoos und dem Auswaschen des Test-Shampoos erfindungsgemäßes Produkt gemäß Ausführungsbeispiel 4 (0,2 ml) aufgetragen und 60 Sekunden einmassiert. Anschließend wurden die Strähnen mit den Fön getrocknet. Die Hitzebehandlung erfolgte wie oben beschrieben. Die Haarsträhnen wurden dann mithilfe der High Pressure Differential Scanning Calorimetry (HPDSC Measurement) untersucht. Hierbei bedeuten höhere Werte bessere α-Keratin-Strukturen und damit intakteres Keratin. Das Ergebnis ist in Abbildung 3 dargestellt. Es wird deutlich, dass die behandelten Haarsträhnen (Code 10) höhere Werte aufweisen als die unbehandelten Haare (Code 05), damit ist gezeigt, dass durch das erfindungsgemäße Produkt ein Hitzeschutz für die Haarsträhnen vorhanden war.

Auch Gegenstand der vorliegenden Erfindung ist die Verwendung einer Zubereitung gemäß wenigstens einem der nachstehenden Ansprüche zur Verbesserung der Nasskämmbarkeit.

Ebenso Gegenstand der vorliegenden Erfindung ist die Verwendung einer Zubereitung gemäß wenigstens einem der nachstehenden Ansprüche, um die Haare mit einem Hitzeschutz auszustatten.

### Beispiele:

Die Beispielrezepturen sollen die Erfindung verdeutlichen, ohne sie einzuschränken. Die Gewichtsangaben beziehen sich auf Gewichtsprozentangaben und Aktivgehalte, sofern nicht anders angegeben.

| **INCI-Bezeichnung** | **1** | **2** | **3** | **4** | **5** |
|---|---|---|---|---|---|
| Aqua | add 100 | add 100 | add 100 | add 100 | add 100 |
| Distearoylethyl Hydroxyethylmonium Methosulfate | 1.75 | 1.05 | 0.525 | 0.7 | 1.4 |
| Cetrimonium Chloride | 0.25 | | | 0.4 | |
| Palmitamidopropyltrimonium Chloride | | 0.18 | | 0.3 | 0.24 |
| Guar Hydroxypropyltrimonium Chloride | | | | 0.065 | 0.87 |
| PEG/PPG-17/18 Dimethicone | | | 0,75 | 1 | |
| VP/VA Copolymer | 3 | 2 | 2.5 | 1 | 2 |
| Cetearyl Alcohol | 0,4 | 1 | 1,25 | 1,8 | 2 |
| Polyquaternium-37 | 0.85 | 1.105 | 1.19 | 0.85 | 1.0625 |
| Parfüm | 0,4 | 0,2 | 0,4 | 0,1 | 0,3 |
| Citric Acid | q.s.* | q.s.* | q.s.* | q.s.* | q.s.* |

| | | | | | |
|---|---|---|---|---|---|
| q.s.* alle Ausführungsbeispiele wurden mit Citronensäure auf einen pH-Wert von 4,6 eingestellt, | | | | | |

## Patentansprüche

1. Kosmetische Haar-Zubereitung, enthaltend
- eine Kombination quaterärer Ammoniumverbindungen ausgewählt aus
• wenigstens einem kationischen Tensid in Form eines Esterquats und
• wenigstens einem kationischen Polymer gemäß folgender Strukturformel 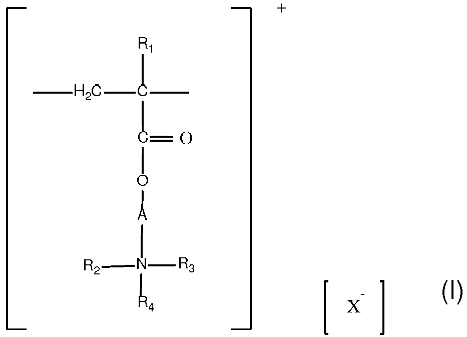 wobei R₁ -CH₃, A -(CH₂)₂, und R₂, R₃ und R₄ jeweils -CH₃ und X⁻ Cl⁻ entsprechen und
- wenigstens ein nichtionisches fixierendes Polymer, enthaltend Vinylpyrolidone-Monomere.

2. Zubereitung gemäß Anspruch 1 **dadurch gekennzeichnet, dass** die Zubereitung nach dem Auftragen auf das menschliche Kopfhaar nicht wieder ausgespült wird (leave-on-Zubereitung).

3. Zubereitung gemäß Anspruch 1 und/oder 2 **dadurch gekennzeichnet, dass** die Zubereitung in Form einer Emulsion vor, insbesondere einer O/W-Emulsion vorliegt.

4. Zubereitung gemäß wenigstens einem der vorstehenden Ansprüche **dadurch gekennzeichnet, dass** die Zubereitung Wasser mit einem Gehalt von 75 bis 98 Gew.-%, besonders bevorzugt 80 bis 95 Gew.-% enthält, bezogen auf das Gesamtgewicht der Zubereitung.

5. Zubereitung gemäß wenigstens einem der vorstehenden Ansprüche **dadurch gekennzeichnet, dass** das wenigstens eine Esterquat ausgewählt wird aus Esterquats mit folgender allgemeiner Strukturformel: 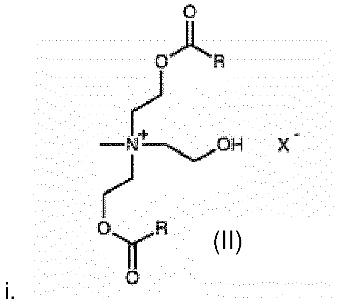 wobei R ein organischer Rest mit 10 bis 30, bevorzugt 12 bis 24 Kohlenstoffatomen ist und X⁻ das Gegenion ist, ausgewählt aus der Gruppe Chlorid, Bromid und Methosulfat.

6. Zubereitung gemäß Anspruch 5 **dadurch gekennzeichnet, dass** der organische Rest ein substituierter oder unsubstituierter, verzweigter oder unverzweigter, optional ungesättigter Alkylrest ist, bevorzugt ein linearer gesättigter Alkylrest mit 14 bis 20 Kohlenstoffatomen.

7. Zubereitung gemäß wenigstens einem der vorstehenden Ansprüche **dadurch gekennzeichnet, dass** das wenigstens eine Esterquat ausgewählt wird aus der Gruppe Dipalmitoylethyl Hydroxyethylmonium Methosulfate und/oder Distearoylethyl Hydroxyethylmonium Methosulfate, insbesondere Distearoylethyl Hydroxyethylmonium Methosulfate.

8. Zubereitung gemäß wenigstens einem der vorstehenden Ansprüche **dadurch gekennzeichnet, dass** das wenigstens eine Esterquat ist mit einem Gehalt von 0,01 bis 10 Gew.-%, bevorzugt 0,1 bis 7,0 Gew.-% und besonders bevorzugt 0,2 bis 5,0 Gew.-% enthalten ist, bezogen auf das Gesamtgewicht der Zubereitung und bezogen auf den Aktivgehalt.

9. Zubereitung gemäß wenigstens einem der vorstehenden Ansprüche **dadurch gekennzeichnet, dass** das kationische Polymer gemäß Strukturformel (I) Polyquaternium-37 ist.

10. Zubereitung gemäß wenigstens einem der vorstehenden Ansprüche **dadurch gekennzeichnet, dass** das wenigstens eine kationische Polymer gemäß Strukturformel (I) mit einem Gehalt von 0,1 bis 8 Gew.-%, bevorzugt 0,2 bis 7.0 Gew.-% und besonders bevorzugt 0,4 bis 5,0 Gew.-% enthalten ist, bezogen auf das Gesamtgewicht der Zubereitung und bezogen auf den Aktivgehalt.

11. Zubereitung gemäß wenigstens einem der vorstehenden Ansprüche **dadurch gekennzeichnet, dass** das wenigstens eine nichtionische fixierende Polymer enthaltend Vinylpyrolidone-Monomere ausgewählt wird aus der Gruppe Homopolymere des Vinylpyrolidons und/oder der Gruppe der Copolymere des Vinylpyrrolidons wie Copolymere aus Vinylpyrrolidon und Vinylacetat (VP/VA), Terpolymere aus Vinylpyrrolidon, Vinylacetat und Propionat, Copolymere aus Vinylpyrrolidon und Dimethylaminoethylmethacrylat, Terpolymere aus Vinylcaprolactam, Vinylpyrrolidon und Dimethylaminomethacrylat.

12. Zubereitung gemäß wenigstens einem der vorstehenden Ansprüche **dadurch gekennzeichnet, dass** das wenigstens eine nichtionische, fixierende Polymer ausgewählt wird aus Copolymeren aus Vinylpyrolidone und Vinylacetat.

13. Zubereitung gemäß wenigstens einem der vorstehenden Ansprüche **dadurch gekennzeichnet, dass** das wenigstens eine nichtionische fixierende Polymer, enthaltend Vinylpyrolidone-Monomere ist in der erfindungsgemäßen Zubereitung mit einem Gehalt von 0,01 bis 10,0 Gew.-%, bevorzugt 0,1 bis 7,0 Gew.-% und besonders bevorzugt 0,2 bis 5,0 Gew.-% enthalten ist, bezogen auf das Gesamtgewicht der Zubereitung und bezogen auf den Aktivgehalt.

14. Zubereitung gemäß wenigstens einem der vorstehenden Ansprüche **dadurch gekennzeichnet, dass** zusätzlich wenigstens eine Ölkomponente enthalten ist, insbesondere ausgewählt aus der Gruppe der gesättigten oder ungesättigten, verzweigten oder unverzweigten Alkohole und/oder aus der Gruppe der Fettsäuretriglyceride, bevorzugt aus der Gruppe der Triglycerinester gesättigter und/oder ungesättigter, verzweigter und/oder unverzweigter Alkancarbonsäuren einer Kettenlänge von 8 bis 24, insbesondere 12 bis 18 C-Atomen.

15. Zubereitung gemäß Anspruch 14 **dadurch gekennzeichnet, dass** die wenigstens eine Ölkomponente wenigstens ein gesättigter und unverzweigter Alkohol mit einer Kettenlänge von 10 bis 24 Kohlenstoffatomen ist, bevorzugt wenigstes ein Alkohol aus der Gruppe Stearylalkohol, Cetylalkohol und/oder der Mischform Cetearylalkohol.

16. Zubereitung gemäß wenigstens einem der vorstehenden Ansprüche **dadurch gekennzeichnet, dass** die wenigstens eine Ölkomponente mit einem Gehalt von 0,1 bis 10,0 Gew.-%, bevorzugt 0,2 bis 6,0 Gew.-% und besonders bevorzugt 0,25 bis 4,0 Gew.-% enthalten ist, bezogen auf das Gesamtgewicht der Zubereitung.

17. Zubereitung gemäß wenigstens einem der vorstehenden Ansprüche **dadurch gekennzeichnet, dass** zusätzlich wenigstens eine Silikonverbindung, bevorzugt wenigstens ein Silikontensid, besonders bevorzugt PEG/PPG-18/18 Dimethicone enthalten ist.

18. Zubereitung gemäß wenigstens einem der vorstehenden Ansprüche **dadurch gekennzeichnet, dass** die wenigstens eine Silikonverbindung mit einem Gehalt von 1,0 bis 7,0 % Gew.-%, bevorzugt 0,2 bis 5,0 % Gew.-% und besonders bevorzugt 0,5 bis 3,0 % Gew.-% enthalten ist, bezogen auf das Gesamtgewicht der Zubereitung und bezogen auf den Aktivgehalt.

19. Zubereitung gemäß wenigstens einem der vorstehenden Ansprüche **dadurch gekennzeichnet, dass** zusätzlich wenigstens ein weiteres kationisches Tensid, das verschieden von Esterquats ist, enthalten ist, bevorzugt ausgewählt aus der Gruppe der Amidoamine und/der der Gruppe der primären Ammoniumsalze.

20. Zubereitung gemäß Anspruch 19 **dadurch gekennzeichnet, dass** das wenigstens eine Amidoamin ausgewählt wird aus der Gruppe Stearylamidopropyltrimmonium Chloride, Ricinoleamidopropyltrimonnium Chloride und/oder Palmitamidopropyltrimonium Chloride; bevorzugt Palmitamidopropyltrimonium Chlorid.

21. Zubereitung gemäß Anspruch 19 **dadurch gekennzeichnet, dass** das wenigstens eine primäre Ammoniumsalz gewählt wird aus der Gruppe Cetyltrimethylammoniumchlorid (Cetrimonium Chlorid), Stearyltrimethylammoniumchlorid und/oder Behentrimoniumchlorid, bevorzugt Cetrimonium Chlorid.

22. Zubereitung gemäß wenigstens einem der vorstehenden Ansprüche **dadurch gekennzeichnet, dass** das wenigstens eine weitere kationische Tensid mit einem Gehalt von 0.1 bis 4 Gew.-%, bevorzugt 0.2 bis 2 Gew.-% enthalten ist, bezogen auf das Gesamtgewicht der Zubereitung und bezogen auf den Aktivgehalt.

23. Zubereitung gemäß wenigstens einem der vorstehenden Ansprüche **dadurch gekennzeichnet, dass** zusätzlich wenigstens ein weiteres kationische Polymer, das verschieden ist von kationischen Polymeren der Strukturformel (I) enthalten ist, bevorzugt ausgewählt aus der Gruppe Polyquaternium 7, Polyquaternium 10 und/oder Guar Hydroxypropyltrimonium Chloride, insbesondere bevorzugt Guar Hydroxypropyltrimonium Chloride.

24. Zubereitung gemäß wenigstens einem der vorstehenden Ansprüche **dadurch gekennzeichnet, dass** das wenigstens eine weitere kationische Polymer mit einem Gehalt von 0,01 bis 1,0 Gew.-%, bevorzugt 0,05 bis 0,75 Gew.-% vorliegt, bezogen auf das Gesamtgewicht der Zubereitung und bezogen auf den Aktivgehalt.

25. Verwendung einer Zubereitung gemäß wenigstens einem der vorstehenden Ansprüche zur Verbesserung der Nasskämmbarkeit.

26. Verwendung einer Zubereitung gemäß wenigstens einem der vorstehenden Ansprüche um die Haare mit einem Hitzeschutz auszustatten.
